# EUROPEAN PATENT SPECIFICATION

(11) **EP 2 061 386 B1**
(45) Date of publication and mention of the grant of the patent: **27.10.2010**
(21) Application number: 07804100.1
(22) Date of filing: 30.08.2007
(51) Int. Cl.: A61B 17/34, A61B 18/14

(54) **SURGICAL INSTRUMENT**
CHIRURGISCHES INSTRUMENT
INSTRUMENT CHIRURGICAL

(30) Priority: 07.09.2006 GB 0617599
(43) Date of publication of application: 27.05.2009
(73) Proprietor: GYRUS MEDICAL LIMITED, Cardiff CF3 0LT (GB)
(72) Inventor: JENKINS, Andrew E, Mid Glamorgan CF41 7AW (GB); BATCHELOR, Kester J., Minneapolis, Minnesota 55416 (US); EBBUTT, Julian Mark, Cardiff CF3 2TA (GB)
(74) Representative: Pratt, David Martin
(86) International application number: PCT/GB2007/003292
(87) International publication number: WO 2008/029092

(56) References cited:
- EP-A- 0 424 002
- EP-A- 0 542 428
- EP-A- 0 696 459
- WO-A-02/30305
- WO-A-94/01149
- WO-A-96/39958
- WO-A-99/42152
- DE-A1- 4 312 147
- US-A- 5 342 315
- US-B1- 6 231 591

## Description

This invention relates to a surgical instrument, and in particular to a bipolar electrosurgical instrument for use in the bulk removal of tissue, as in a laparoscopic hysterectomy. The invention also relates to a surgical instrument provided with a sealing assembly for preventing the escape, through the instrument, of an inert gas which is pumped to an operation site associated with the surgical instrument.

In a laparoscopic hysterectomy, the body of the uterus is resected from the stump or fundus, and then removed from the operative site. To enable the uterus to be removed through a limited surgical opening, it is desirable to morcellate it into relatively smaller pieces of tissue, which are easier to remove. The present invention relates to an instrument and method for morcellating and removing a uterus.

US patent specifications 5,957,884, 6,007,512 and 6,036,681 describe examples of morcellating devices in which an element carrying an electrode is rotated in order to cause the morcellation of tissue. This rotation of the electrode necessitates a mechanical drive arrangement, which increases the complexity of the instrument.

The specification of International patent application PCT/GB2005/001922 seeks to provide a simpler, and hence more reliable, arrangement for the bulk removal of tissue, and provides a combination of a device for morcellating tissue within a body cavity of a patient and a tissue-pulling device. The morcellating device comprises a stationary tube having a distal end portion, the tissue-pulling device being locatable within the tube. The combination includes a bipolar electrosurgical electrode assembly including first and second electrodes, the first electrode being located at the distal end of the tube, such that, when an electrosurgical cutting voltage is applied to the electrode assembly, the tissue-pulling device can be moved to pull tissue against the distal end of the tube to form a core of severed tissue within the tube, and further moved in order to remove the severed tissue from the body cavity of the patient.

The morcellating device of this known system operates under an inert gas such as CO₂, with the current flow from the active electrode to the return electrode being via tissue. The inert gas is introduced to the operation site either through the morcellating instrument itself, or via a separate endoscopic instrument. A disadvantage of the known instrument is that the inert gas can pass from the operation site, through the stationary tube to exit from the proximal end of the tube where the tissue-pulling device passes through that end of the tube. Escape of the inert gas is particularly of concern when the tissue-pulling device is operated, as the relative movement between the tissue-pulling device and the stationary tube facilitates the escape of inert gas from the interior of the tube. The escape of inert gas is undesirable, particularly where it leads to surges in the flow of inert gas supplied to the surgical site.

It is known to provide a seal at the proximal end of the tube to reduce the escape of inert gas, and typically this is constituted by a duckbill valve. The disadvantage of such a valve is that, as morcellated tissue is withdrawn through the tube, it tends to engage the edges of the duckbill valve, thereby at least partially opening the valve, and hence leading to an undesirable escape of inert gas. The engagement of morcellated tissue with such a valve also impedes proximal movement of the tissue, and risks tearing of the valve and/or loss of grip of the tissue.

A further device of the art is disclosed in EP 0542428 A1.

An aim of the invention is to provide an improved seal for the proximal end of a morcellating surgical instrument.

The present invention provides a surgical system comprising a housing, a surgical instrument and a sealing assembly, the surgical instrument being locatable within the housing, the system being usable for surgery in which a surgical operation site is supplied with pressurised gas, the housing having a proximal end locatable outside the body of a patient, the surgical instrument having a distal end locatable at the surgical operation site, and the sealing assembly being provided at the proximal end of the housing to seal the housing with respect to the proximal end portion of the surgical instrument to prevent gas escaping from the surgical operation site through the proximal end of the housing, wherein the sealing assembly is constituted by a diaphragm whose peripheral edge portions are fixed to the housing, the central portion of the diaphragm being formed with at least one slit through which the surgical instrument passes, the sealing assembly being such that movement of the surgical instrument relative to the housing causes the diaphragm to move between first and second operating positions in which the slit faces respectively towards and away from the proximal end of the housing, and such that the edges of the slit sealingly engage with the surgical instrument in the first and second operating positions, and in all positions therebetween, wherein the slit in the central portion is longitudinally offset from the circumferential edge portion of the diaphragm when the sealing assembly is in its first and second operating positions, the offset being in one longitudinal direction in the first operating position and in the other longitudinal direction in the second operating position, the second longitudinal direction being opposite to the first longitudinal direction, and wherein the central portion of the diaphragm is substantially dome-shaped, and is connected to the circumferential edge portion thereof by a thin annular section. The thickness of the thin annular section is less than that of the central portion.

When the seal is in its second operating position, it toggles such that the opening segments of the seal face the proximal end of the surgical instrument, thereby offering a proximally-tapering configuration to the surgical instrument (or to tissue associated with the surgical instrument), which minimises impediment to proximal movement, minimises the escape of gas, and reduces the chance of the seal tearing.

Preferably, a tubular member constitutes the housing, the tubular member having a distal end locatable at the surgical operation site. The housing may merely be a tubular element allowing the insertion and withdrawal of other surgical instruments, but conceivably the housing may be a part of a surgical instrument in its own right, such as a morcellator or other surgical instrument.

In a preferred embodiment, the system further comprises an extension piece at the proximal end of the tubular member, the proximal end of the extension piece having a circular cross-section, and the diaphragm being fixed with its circumferential edge portion within the circumferential proximal end portion of the extension piece. The extension piece may be fixed to the proximal end of the tubular member.

Preferably, the cross-sectional area of the proximal end of the extension piece is larger than the proximal end portion of the tubular member.

The tubular member may have a circular cross-section.

In a preferred embodiment, the sealing assembly is such that the dome-shaped central portion faces respectively towards and away from the proximal end of the tubular member when the diaphragm is in its first and second operating positions.

Advantageously, the cross-section of the diaphragm is reduced in the region of the slit, as compared to at least one other region of the diaphragm. In this case, the diaphragm may comprise an inner portion having a first cross-sectional thickness, an outer portion comprising a second cross-sectional thickness, the first cross-sectional thickness being less than the second cross-sectional thickness.

In a preferred embodiment, the central portion of the diaphragm is constituted by a relatively thin laminar member. The central portion of the diaphragm may itself have a changing cross-sectional thickness, either tapered or stepped in construction. This helps to provide a more conformal fit around the surgical instrument, creating a better gas-tight seal and presenting less frictional resistance to the movement of the instrument. This construction can be particularly effective when different surgical instruments, having shafts of different diameters, are to be used in conjunction with the seal.

Advantageously, the thin laminar member is positioned centrally within a relatively thick circumferential edge portion of the diaphragm.

Preferably, the thin laminar member is connected to the circumferential edge portion by an annular section that is thinner than the laminar member. This thinner annular section prevents any location forces being transferred to the diaphragm edges during construction, and constitutes a deformation absorption area, which preferentially allows the seal to invert.

Advantageously, the annular connecting section is connected to an outer circumferential edge portion of the laminar member that has a thickness greater than the central portion of the laminar member, thereby providing structural rigidity to react against abdominal pressure when no device is inserted, and to encourage close mating of the thinner region with an inserted surgical instrument.

Preferably, the slit is symmetrically positioned with respect to the centre of the diaphragm, and the slit is a cross-shaped slit. The advantage of this is that, if the surgical instrument is not positioned centrally within the tubular member, it will tend to engage with only one of the triangular flaps defined by the cross-shaped slit, thereby limiting the loss of sealing capacity provided by the diaphragm.

The provision of this laminar member being a cross-shaped slit ensures that, if a tissue particle adheres to the exterior of the surgical instrument one or more of the triangular flaps defined by the slit can closely engage around such a particle, thereby maintaining the sealing action.

The sealing assembly may further comprise an annular seal positioned within the housing proximally of the diaphragm and engageable with the surgical instrument.

Advantageously, the annular seal is positioned at the proximal end of the extension piece.

In a preferred embodiment, the surgical instrument is constituted by a tissue-pulling device.

In this case, the system further comprises a morcellating device positioned at the distal end of the tubular member.

Preferably, the morcellating device is constituted by a bipolar electrosurgical electrode assembly including first and second electrodes, the first electrode being located at the distal end of the tubular member such that, when an electrosurgical cutting voltage is applied to the electrode assembly, the tissue-pulling device can be moved to pull tissue against the distal end of the tubular member to form a core of severed tissue within the tubular member, and can be further moved in order to remove severed tissue from the surgical operation site.

The surgical instrument may be constituted by an insufflation tube or a trocar.

The invention also provides a housing, a surgical instrument and a sealing assembly, the surgical instrument being locatable within the housing, the system being usable for surgery in which a surgical operation site is supplied with pressurised gas, the housing having a proximal end locatable outside the body of a patient, the surgical instrument having a distal end locatable at the surgical operation site, and the sealing assembly being provided at the proximal end of the housing to seal the housing with respect to the proximal end portion of the surgical instrument to prevent gas escaping from the surgical operation site through the proximal end of the housing, wherein the sealing assembly is constituted by a diaphragm whose peripheral edge portions are fixed to the housing, the central portion of the diaphragm being formed with at least one slit through which the surgical instrument passes, the sealing assembly being such that movement of the surgical instrument relative to the housing causes the diaphragm to move between first and second operating positions in which the slit faces respectively towards and away from the proximal end of the housing, and such that the edges of the slit sealingly engage with the surgical instrument in the first and second operating positions, and in all positions therebetween, wherein the slit in the central portion is longitudinally offset from the circumferential edge portion of the diaphragm when the sealing assembly is in its first and second operating positions, the offset being in one longitudinal direction in the first operating position and in the other longitudinal direction in the second operating position, the second longitudinal direction being opposite to the first longitudinal direction, and wherein the central portion of the diaphragm is constituted by a relatively thin laminar member positioned centrally within a relatively thick circumferential edge portion of the diaphragm, the thin laminar member being connected to the circumferential edge portion by an annular section that is thinner than the laminar member, and is so thin that it prevents any force acting on the central portion from being transferred to the peripheral edge portion.

The invention will now be described in more detail, by way of example, with reference to the drawings, in which;
Figure 1 is a schematic side view of a morcellating system constructed in accordance with the invention;
Figure 2 is a schematic sectional view of a part of the electrosurgical instrument of the system of Figure 1;
Figure 3 is a view similar to that of Figure 2, illustrating a first operational position of a seal of the electrosurgical instrument;
Figure 4 is a view similar to that of Figure 3, illustrating a second operational position of the seal;
Figure 5 is a perspective view illustrating the action of the seal;
Figure 6 is a perspective view of a modified form of seal;
Figure 7 is a sectional view of the seal of Figure 6;
Figure 8 is a sectional view of another modified form of seal; and
Figure 9 and 10 are sectional views of other modified forms of seal.

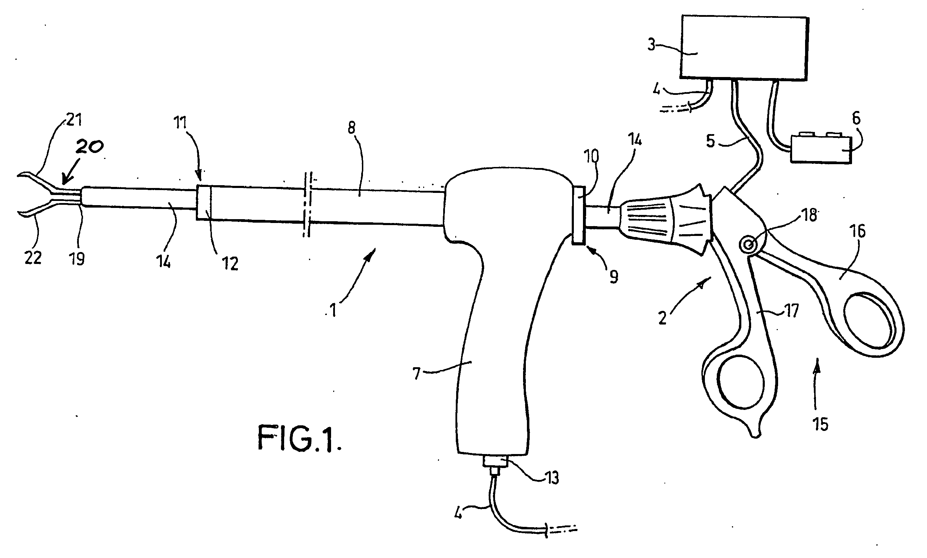

Referring to the drawings, Figure 1 shows a morcellating system comprising a morcellating device 1, a tissue-pulling device 2, and an electrosurgical generator 3. The generator 3 is connected to the morcellating device 1 by means of a cable 4, and to the tissue-pulling device 2 by means of a cable 5. The generator 3 is controlled by means of a footswitch 6.

The morcellating device 1 comprises a handle 7 and a cylindrical tube 8. The cylindrical tube 8 is hollow, and defines a lumen therein. The proximal end 9 of the tube 8 extends from the handle 7 as shown at 10, and the distal end 11 of the tube is provided with an electrosurgical electrode assembly 12. The electrosurgical electrode assembly 12 comprises an active tissue-cutting electrode (not shown), and an insulating member (not shown), both extending around the circumference of the tube 8. The insulating member separates the active electrode from the remainder of the tube 8, which acts as a return electrode.

The tube 8 is connected to one pole of the generator 3, via the cable 4 and a connector 13. The active electrode extends around the entire circumference of the tube 8, and is connected to the other pole of the generator 3, via the cable 4, the connector 13 and additional wiring (not shown). In this way, the active and return electrodes constitute the bipolar electrode assembly 12, which, when energised by the generator 3, is capable of cutting tissue coming into contact with the distal end 11 of the tube 8.

The tissue-pulling device 2 comprises a tubular shaft 14, at the proximal end of which is a scissors-type handle mechanism 15, having a first handle 16 and a second handle 17. The second handle 17 is pivotable with respect to the first handle 16, about a pivot pin 18. Pivoting of the second handle 17 causes longitudinal movement of a push rod 19 extending through the shaft 14 to the distal end thereof.

A jaw assembly 20 is provided at the distal end of the shaft 14, the jaw assembly having a first jaw member 21 and a second jaw member 22 movable between open and closed positions by the movement of the push rod 19. The tissue-pulling device 2 is manually translatable in a longitudinal manner within the lumen of the morcellating device 1 by means of slidable guide members (not shown) supporting the shaft 14 of the tissue-pulling device within the tube 8. The jaw members 21 and 22 are electrically connected to the shaft 14, and the shaft is electrically connected, via the cable 5 and a connector (not shown), to the generator 3. The shaft 14 is connected to the same pole of the generator 3 as the return electrode constituted by the tube 8.

The operation of the morcellating system is as follows. The tube 8 of the morcellating device 1 is inserted into the body of a patient, either directly or through a trocar (not shown), and brought into position adjacent to the tissue to be removed (typically a resected uterus in the case of a laparoscopic hysterectomy). The tissue-pulling device 2 is then inserted through the lumen of the morcellating device 1. The handle 17 is operated to open the jaw assembly 20, and the tissue-pulling device 2 is manoeuvred so that tissue from the uterus is located between the jaw members 21 and 22. The handle 17 is then operated to close the jaw assembly 20, grasping tissue therein.

The surgeon operates the footswitch 6 to operate the generator 3 so that an electrosurgical cutting voltage is supplied between the tissue-cutting electrode and the return electrode. As mentioned previously, the push rod 19 and the jaw assembly 20 are also electrically connected to the same pole of the generator 3 as the tube 8, and so both the tube and the jaw assembly constitute the return electrode. With tissue firmly grasped in the jaw assembly 20, the device 2 is slowly withdrawn from the tube 8, pulling the tissue against the distal end of the tube and the tissue-cutting electrode. As the tissue contacts the tissue-cutting electrode, it is vaporised, allowing the device 2 to be withdrawn further into the tube 8. In this way, a cylindrical core of tissue is formed in the tube 8, the tissue being withdrawn though the proximal end 9 of the morcellating device 1 (which remains outside the body of the patient) for disposal.

The tissue-pulling device 2 can then be re-inserted into the tube 8 such that a further core of tissue can be removed from the body of the patient. By repeating this process, large quantities of tissue can be removed from the patient in a relatively short time, such that the entire uterus can be removed, if necessary, while still employing a laparoscopic approach.

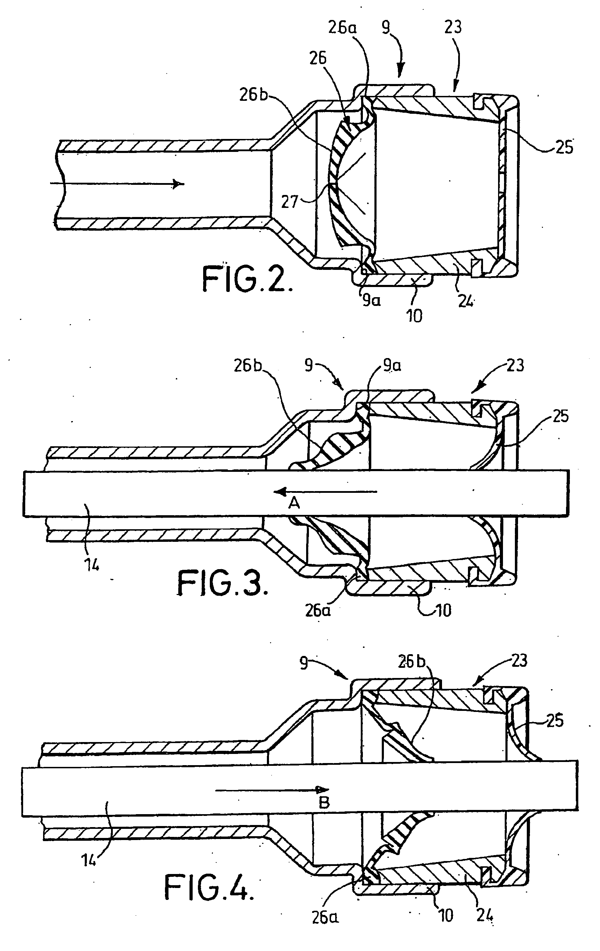

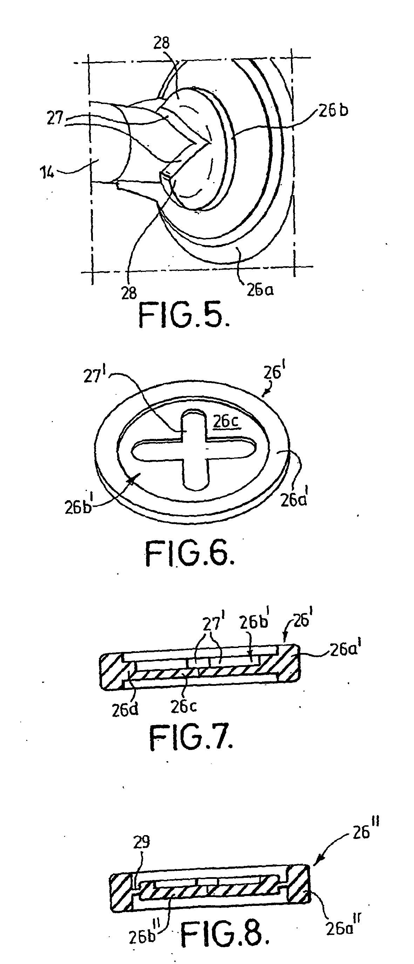

The proximal end 9 of the tube 8 is shown in greater detail in Figure 2, this figure omitting the shaft 14 of the tissue-pulling device 2. The extension 10 of the proximal end 9 of the tube is provided with a sealing assembly 23 constituted by a generally cylindrical body 24 a rear (instrument) seal 25 and a seal 26. Both rear seal 25 and the seal 26 engage, in use, around the outer periphery of the tubular shaft 14 (see Figure 3). The rear seal 25 has an internal diameter of 10mm or less to complement the outer diameter of the shaft 14, and is made of a plastics material such as polypropylene. The seal 26 is made of a silicone rubber material, and has a circumferential edge portion 26a and a central portion 20b, the edge portion being gripped between the cylindrical body 24 and a shoulder 9a formed in the proximal end 9 of the tube 8. The central portion 26b of the seal 26 is substantially dome-shaped, and is formed with central cross-shaped slit 27 (not shown fully in Figure 2, but similar to the slit 27' of the seal 26' of Figures 5 and 6 - to be described below).

Figure 3 shows the seal 26 of the assembly 23 in engagement with the shaft 14 of the tissue-pulling device 2, and shows the position of the seal 26 as the tissue-pulling device moves forwardly with respect to the tube 8 so that the shaft moves in the direction of the arrow A in Figure 3. In this position (which is shown in greater detail in Figure 5), the four flaps 28 defined by the cross-shaped slit 27 open up and engage around the circumference of the shaft 14, thereby forming an effective gas-tight seal.

When the shaft 14 is moved in the opposite direction B (see Figure 4), frictional engagement between the shaft and the seal 26 moves the dome-shaped seal portion 26b in such a manner as to reverse the shape of the dome so that the flaps 28 extend towards the proximal end of the tube 18, thereby forming an effective seal against the outer circumference of the shaft. Thus, respective of the position of the shaft 14 with respect to the tube 8, the seal 26 closely engages the shaft to provide an effective gas-tight seal there against.

The advantage of the cross-shaped slit 27 is that an effective seal is provided even if the shaft 14 is not positioned centrally with respect to the tube 8, that is to say if the axis of the shaft is not coincident with the centre of the cross-shaped slit. Thus; in such a situation, only one of the triangular flaps 28 will tend to open as the shaft 14 moves relative to the seal 26, thereby limiting any gap between the seal and the shaft, and so limiting gas leakage.

Figures 6 and 7 show a modified form of seal 26', this seal having a central portion 26b' which is substantially in the same plane as its peripheral edge portion 26a'. The central portion 26b', which contains a cross-shaped slit 27' is provided in a thinner (laminar) central portion 26c of the seal 26'. This results in a more flexible scaling member which can engage more easily around tissue particles that adhere to the shaft 14, thereby improving the sealing action. The thinner central portion 26 is connected to the peripheral edge portion 26c' by an annular portion 26d of intermediate thickness. The portions 26c and 26d meet at a right-angled shoulder 26c. Alternatively, the shoulder would be angled. Fig. 7 does not form part of the invention.

Figure 8 shows a modification of the seal shown in Figures 6 and 7, this seal 26" being substantially identical to the seal 26', but having its central portion 26b" connected to its peripheral edge portion 26a" by a very thin annular strip 29. The annular strip 29 prevents any forces acting on the central portion 26b" as a result of the movement of the shaft 14 being transferred to the peripheral edge portion 26a".

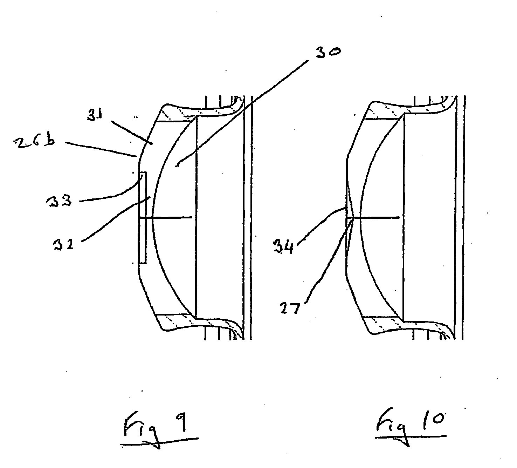

Figures 9 and 10 show further modifications to the seal shown in Figures 6 and 7. In Figure 9, the central portion 26b is generally dome-shaped as shown at 30, and has a thicker outer section 31, transitioning to a thinner inner section 32 by means of a shoulder 33. In Figure 10, the central portion does not have a shoulder 33, but tapers smoothly from a thicker outer section to a thinner central section at the slit 27, the taper being shown generally at 34. Each of these arrangements helps to maintain an effective gas-tight seal, regardless of whether the shaft 14 has a relatively large diameter (typically 10 mm) or a relatively small diameter (typically 5 mm).

It will be apparent that modifications could be made to the sealing assemblies described above. In particular, the cross-shaped slit could be replaced by a slit of a different configuration, for example a linear slit. Although a linear slit would not provide sealing properties as good as a cross-shaped slit, it will provide sufficient sealing capabilities, particularly if the slit is formed in a thin portion of soft flexible plastics material. It would, of course, also be possible to provide a cross-shaped slit having a different number of arms, thereby providing a different number of triangular flaps for sealing against the shaft of the tissue-pulling device. For example slits having three, five or six arms would provide adequate sealing.

The sealing system described above could also be used with other types of surgical instruments which are used at operation sites to which an inert gas is pumped, and which are disadvantaged by the escape of gas from the proximal ends thereof. For example, the seal assembly shown in Figures 2 and 3 could be used as an insufflation seal system. In that case, the shaft 14 would be replaced by an insufflation supply tube. In all other respects, the seal would work in exactly the same way.

Another possibility would be to use the seal system described above with a trocar, in which case the trocar would replace the shaft 14 shown in Figures 2 to 4. Here again, the seal would work in exactly the same way.

In either of these alternatives, the surgical instrument would be further modified by the addition of taps, ports and other such connections that are required for the correct operation of a trocar or an insufflation instrument.

## Claims

1. A surgical system comprising a housing (8, 24), a surgical instrument (14, 21, 22) and a sealing assembly (23), the surgical instrument being locatable within the housing (3, 24), the system being usable for surgery in which a surgical operation site is supplied with pressurised gas, the housing having a proximal end locatable outside the body of a patient, the surgical instrument having a distal end locatable at the surgical operation site, and the sealing assembly (23) being provided at the proximal end (9) of the housing to seal the housing with respect to the proximal end portion of the surgical instrument (14, 21, 22) to prevent gas escaping from the surgical operation site through the proximal end of the housing, wherein the sealing assembly (23) is constituted by a diaphragm (26) whose peripheral edge portions (26a) are fixed to the housing, the central portion (26b) of the diaphragm being formed with at least one slit (27) through which the surgical instrument passes, the sealing assembly (23) being such that movement of the surgical instrument (14, 21, 22) relative to the housing (8, 24) causes the diaphragm (26) to move between first and second operating positions in which the slit (27) faces respectively towards and away from the proximal end (9) of the housing, and such that the edges of the slit sealingly engage with the surgical instrument in the first and second operating positions, and in all positions therebetween, wherein the slit (27) in the central portion (26b) is longitudinally offset from the circumferential edge portion (26a) of the diaphragm when the sealing assembly (23) is in its first and second operating positions, the offset being in one longitudinal direction in the first operating position and in the other longitudinal direction in the second operating position, the second longitudinal direction being opposite to the first longitudinal direction, and wherein the central portion (26b) of the diaphragm is substantially dome-shaped, and is connected to the circumferential edge portion (26a) thereof by a thin annular section.

2. A system as claimed in claim 1, wherein the housing (8, 24) comprises a tubular member (8), and wherein the sealing assembly is such that the dome-shaped central portion (26b) faces respectively towards and away from the proximal end of the tubular member (8) when the diaphragm (26) is in its first and second operating positions.

3. A system as claimed in claim 1 or claim 2, wherein the slit (27) is symmetrically positioned with respect to the centre of the diaphragm (26).

4. A system as claimed in any one of claims 1 to 3, wherein the slit (27) is a cross-shaped slit.

5. A system as claimed in any one of claims 1 to 4 wherein the cross-section of the diaphragm (26) is reduced in the region of the slit (27) as compared to at least one other region of the diaphragm.

6. A system as claimed in any one of claims 1 to 5, wherein the sealing assembly (23) further comprises an annular seal (25) positioned within the housing (8, 24) proximally of the diaphragm (26) and engageable with the surgical instrument (14, 21, 22).

7. A system as claimed in claim 6, wherein the annular seal (25) is positioned at the proximal end of an extension piece (24) at the proximal end of the tubular member (8).

8. A system as claimed in any one of claims 1 to 7, wherein the surgical instrument is constituted by a tissue-pulling device.

9. A system as claimed in claim 8, further comprising a morcellating device positioned at the distal end of the tubular member (8).

10. A system as claimed in claim 9, wherein the morcellating device is constituted by a bipolar electrosurgical electrode assembly including first and second electrodes, the first electrode being located at the distal end of the tubular member such that, when an electrosurgical cutting voltage is applied to the electrode assembly, the tissue-pulling device can be moved to pull tissue against the distal end of the tubular member (8) to form a core of severed tissue within the tubular member, and can be further moved in order to remove severed tissue from the surgical operation site.

11. A surgical system comprising a housing (8, 24), a surgical instrument (14, 21, 22) and a sealing assembly (23), the surgical instrument being locatable within the housing (8, 24), the system being usable for surgery in which a surgical operation site is supplied with pressurised gas, the housing having a proximal end locatable outside the body of a patient, the surgical instrument having a distal end locatable at the surgical operation site, and the sealing assembly (23) being provided at the proximal end of the housing to seal the housing with respect to the proximal end portion of the surgical instrument to prevent gas escaping from the surgical operation site through the proximal end of the housing, wherein the scaling assembly (23) is constituted by a diaphragm (26) whose peripheral edge portions (26a") are fixed to the housing, the central portion (26b") of the diaphragm being formed with at least one slit (27) through which the surgical instrument passes, the sealing assembly (23) being such that movement of the surgical instrument relative to the housing causes the diaphragm (26) to move between first and second operating positions in which the slit faces respectively towards and away from the proximal end of the housing, and such that the edges of the slit sealingly engage with the surgical instrument in the first and second operating positions, and in all positions therebetween, wherein the central portion of the diaphragm is constituted by a thin laminar member (26b") positioned centrally within a thick circumferential edge portion (26a") of the diaphragm, the thin laminar member (26b") being connected to the circumferential edge portion (26a") by an annular section (29) that is thinner than the laminar member (26b") so that it prevents a force acting on the central portion from being transferred to the peripheral edge portion of the diaphragm (26).

12. A system as claimed in claim 11, wherein the diaphragm (26) comprises an inner portion (26b") having a first cross-sectional thickness, an outer portion (26a") comprising a second cross-sectional thickness, the first cross-sectional thickness being less than the second cross-sectional thickness.

## Patentansprüche

1. Chirurgisches System, umfassend ein Gehäuse (8, 24), ein chirurgisches Instrument (14, 21, 22) und eine Dichtungseinheit (23), wobei das chirurgische Instrument in dem Gehäuse (8, 24) in Anordnung gebracht werden kann, das System für eine Operation verwendbar ist, bei der das Operationsfeld mit Druckgas versorgt wird, das Gehäuse ein proximales Ende hat, das außerhalb des Körpers eines Patienten in Anordnung gebracht werden kann, das chirurgische Instrument ein distales Ende hat, das an dem Operationsfeld in Anordnung gebracht werden kann, und die Dichtungseinheit (23) an dem proximalen Ende (9) des Gehäuses vorgesehen ist, um das Gehäuse hinsichtlich des proximalen Endbereichs des chirurgischen Instruments (14, 21, 22) abzudichten und zu verhindern, dass Gas durch das proximale Ende des Gehäuses aus dem Operationsfeld entweicht, wobei die Dichtungseinheit (23) durch eine Membrane (26) gebildet ist, deren Umfangskantenbereiche (26a) an dem Gehäuse fixiert sind, wobei der zentrale Bereich (26b) der Membrane mit zumindest einem Schlitz (27) ausgebildet ist, durch den das chirurgische Element hindurchtritt, wobei die Dichtungseinheit (23) dergestalt ist, dass die Bewegung des chirurgischen Instruments (14, 21, 22) relativ zu dem Gehäuse (8, 24) bewirkt, dass sich die Membrane (26) zwischen der ersten und der zweiten Betriebsposition bewegt, in der der Schlitz (27) dem proximalen Ende (9) des Gehäuses zugekehrt und von dem proximalen Ende des Gehäuses abgekehrt ist, und dass die Schlitzkanten in der ersten und in der zweiten Betriebsposition und in sämtlichen Zwischenpositionen unter Abdichtung an dem chirurgischen instrument angreifen, wobei der Schlitz (27) in dem zentralen Bereich (26b) von dem Umfangskantenbereich (26a) der Membrane versetzt ist, wenn sich die Dichtungseinheit (23) in ihrer ersten und zweiten Betriebsposition befindet, wobei der Versatz in der ersten Betriebsposition in der einen Längsrichtung und in der zweiten Betriebsposition in der anderen Längsrichtung erfolgt, wobei die zweite Längsrichtung zur ersten Längsrichtung entgegengesetzt ist und wobei der zentrale Bereich (26b) der Membrane im Wesentlichen kuppelförmig ist und durch einen dünnen ringförmigen Abschnitt mit dem Umfangskantenbereich (26a) verbunden ist.

2. System nach Anspruch 1, wobei das Gehäuse (8, 24) ein rohrförmiges Element (8) aufweist und wobei die Dichtungseinheit dergestalt ist, dass der kuppelförmige zentrale Bereich (26b) dem proximalen Ende des rohrförmigen Elements (8) zugekehrt ist und von dem proximalen Ende des rohrförmigen Elements abgekehrt ist, wenn sich die Membrane (26) in ihrer ersten und zweiten Betriebsposition befindet.

3. System nach Anspruch 1 oder 2, wobei der Schlitz (27) hinsichtlich der Mitte der Membrane (26) symmetrisch positioniert ist

4. System nach einem der Ansprüche 1 bis 3, wobei der Schlitz (27) ein kreuzförmiger Schlitz ist.

5. System nach einem der Ansprüche 1 bis 4, wobei der Querschnitt der Membrane (26) in dem Bereich des Schlitzes (27) gegenüber zumindest einem anderen Bereich der Membrane verringert ist.

6. System nach einem der Ansprüche 1 bis 5, wobei die Dichtungseinheit (23) ferner eine Ringdichtung (25) umfasst, die in dem Gehäuse (8, 24) proximal zur Membrane (26) angeordnet ist und die mit dem chirurgischen Instrument (14, 21, 22) in Eingriff gebracht werden kann.

7. System nach Anspruch 6, wobei die Ringdichtung (25) an dem proximalen Ende eines Verlängerungsteils (24) an dem proximalen Ende des rohrförmigen Elements (8) positioniert ist.

8. System nach einem der Ansprüche 1 bis 7, wobei das chirurgische Instrument durch eine Gewebezange gebildet ist.

9. System nach Anspruch 8, ferner umfassend eine Morcellations-Vorrichtung, die an dem distalen Ende des rohrförmigen Elements (8) positioniert ist.

10. System nach Anspruch 9, wobei die Morcellations-Vorrichtung durch eine bipolare elektrochirurgische Elektrodenanordnung mit einer ersten und einer zweiten Elektrode gebildet ist, wobei sich die erste Elektrode an dem distalen Ende des rohrförmigen Elements befindet, so dass die Gewebezange bei Anlegen einer elektrochirurgischen Schnittspannung an die Elektrodenanordnung bewegt werden kann, um Gewebe an das distale Ende des rohrförmigen Elements (8) zu ziehen, um einen Kern abgetrennten Gewebes in dem rohrförmigen Element zu bilden, und dass die Gewebezange weiter bewegt werden kann, um das abgetrennte Gewebe von dem Operationsfeld zu entfernen.

11. Chirurgisches System, umfassend ein Gehäuse (8, 24), ein chirurgisches Instrument (14, 21, 22) und eine Dichtungseinheit (23), wobei das chirurgische Instrument in dem Gehäuse (8, 24) in Anordnung gebracht werden kann, das System für eine Operation verwendbar ist, bei der das Operationsfeld mit Druckgas versorgt wird, das Gehäuse ein proximales Ende hat, das außerhalb des Körpers eines Patienten in Anordnung gebracht werden kann, das chirurgische Instrument ein distales Ende hat, das an dem Operationsfeld in Anordnung gebracht werden kann, und die Dichtungseinheit (23) an dem proximalen Ende (9) des Gehäuses vorgesehen ist, um das Gehäuse hinsichtlich des proximalen Endbereichs des chirurgischen Instruments (14, 21, 22) abzudichten und zu verhindern, dass Gas durch das proximale Ende des Gehäuses aus dem Operationsfeld entweicht, wobei die Dichtungseinheit (23) durch eine Membrane (26) gebildet ist, deren Umfangskantenbereiche (26a) an dem Gehäuse fixiert sind, wobei der zentrale Bereich (26b") der Membrane mit zumindest einem Schlitz (27) ausgebildet ist, durch den das chirurgische Element hindurchtritt, wobei die Dichtungseinheit (23) dergestalt ist, dass die Bewegung des chirurgischen Instruments relativ zu dem Gehäuse bewirkt, dass sich die Membrane (26) zwischen der ersten und der zweiten Betriebsposition bewegt, in der der Schlitz dem proximalen Ende des Gehäuses zugekehrt und von dem proximalen Ende des Gehäuses abgekehrt ist, und dass die Schlitzkanten in der ersten und in der zweiten Betriebsposition und in sämtlichen Zwischenpositionen unter Abdichtung an dem chirurgischen Instrument angreifen, wobei der zentrale Bereich der Membrane durch ein dünnes Lamellenelement (26b") gebildet ist, das zentral innerhalb des dicken Umfangskantenbereichs (26a") der Membrane positioniert ist, wobei das dünne Lamellenelement (26b") durch einen ringförmigen Abschnitt (29), der dünner als das Lamellenelement (26b") ist und so verhindert, dass eine auf den zentralen Bereich wirkende Kraft auf den Umfangskantenbereich der Membrane (26) übertragen wird, mit dem Umfangskantenbereich (26a") verbunden ist.

12. System nach Anspruch 11, wobei die Membrane (26) einen inneren Bereich (26b") aufweist, der eine erste Querschnittdicke hat, wobei ein äußerer Bereich (26a") eine zweite Querschnittdicke aufweist und wobei die erste Querschnittdicke geringer ist als die zweite Querschnittdicke.

## Revendications

1. Système chirurgical comprenant un boîtier (8, 24), un instrument chirurgical (14, 21, 22) et un ensemble d'étanchéité (23), l'instrument chirurgical pouvant être positionné à l'intérieur du boîtier (8, 24), le système pouvant être utilisé pour une chirurgie dans laquelle un site d'opération chirurgicale est alimenté avec du gaz sous pression, le boîtier ayant une extrémité proximale pouvant être positionnée à l'extérieur du corps d'un patient, l'instrument chirurgical ayant une extrémité distale pouvant être positionnée au niveau du site d'opération chirurgicale, et l'ensemble d'étanchéité (23) étant prévu au niveau de l'extrémité proximale (9) du boîtier afin de réaliser l'étanchéité du boîtier par rapport à la partie d'extrémité proximale de l'instrument chirurgical (14, 21, 22) pour empêcher la fuite du gaz du site d'opération chirurgicale par l'extrémité proximale du boîtier, dans lequel l'ensemble d'étanchéité (23) est constitué par un diaphragme (26) dont les parties de bord périphérique (26a) sont fixées sur le boîtier, la partie centrale (26b) du diaphragme étant formée avec au moins une fente (27) à travers laquelle l'instrument chirurgical passe, l'ensemble d'étanchéité (23) étant tel que le mouvement de l'instrument chirurgical (14, 21, 22) par rapport au boîtier (8, 24) amène le diaphragme (26) à se déplacer entre une première et une deuxième position opérationnelle dans lesquelles la fente (27) est respectivement orientée vers et à distance de l'extrémité proximale (9) du boîtier, et de sorte que les bords de la fente se mettent en prise de manière étanche avec l'instrument chirurgical dans les première et deuxième positions opérationnelles, et dans toutes les positions entre elles, dans lequel la fente (27) dans la partie centrale (26b) est décalée de manière longitudinale par rapport à la partie de bord circonférentiel (26a) du diaphragme lorsque l'ensemble d'étanchéité (23) est dans ses première et deuxième positions opérationnelles, le décalage étant dans une direction longitudinale dans la première position opérationnelle et dans l'autre direction longitudinale dans la deuxième position opérationnelle, la deuxième direction longitudinale étant opposée à la première direction longitudinale, et dans lequel la partie centrale (26b) du diaphragme est sensiblement en forme de dôme et est raccordée à sa partie de bord circonférentiel (26a) par une fine section annulaire.

2. Système selon la revendication 1, dans lequel le boîtier (8, 24) comprend un élément tubulaire (8), et dans lequel l'ensemble d'étanchéité est tel que la partie centrale en forme de dôme (26b) est respectivement orientée vers et à distance de l'extrémité proximale de l'élément tubulaire (8) lorsque le diaphragme (26) est dans ses première et deuxième positions opérationnelles.

3. Système selon la revendication 1 ou la revendication 2, dans lequel la fente (27) est positionnée de manière symétrique par rapport au centre du diaphragme (26).

4. Système selon l'une quelconque des revendications 1 à 3, dans lequel la fente (27) est une fente en forme de croix.

5. Système selon l'une quelconque des revendications 1 à 4, dans lequel la section transversale du diaphragme (26) est réduite dans la région de la fente (27), par rapport à au moins une autre région du diaphragme.

6. Système selon l'une quelconque des revendications 1 à 5, dans lequel l'ensemble d'étanchéité (23) comprend en outre un joint d'étanchéité annulaire (25) positionné à l'intérieur du boîtier (8, 24) de manière proximale par rapport au diaphragme (26) et pour se mettre en prise avec l'instrument chirurgical (14, 21, 22).

7. Système selon la revendication 6, dans lequel le joint d'étanchéité annulaire (25) est positionné au niveau de l'extrémité proximale d'une pièce d'extension (24) au niveau de l'extrémité proximale de l'élément tubulaire (8).

8. Système selon l'une quelconque des revendications 1 à 7, dans lequel l'instrument chirurgical est constitué par un dispositif de traction de tissu.

9. Système selon la revendication 8, comprenant en outre un dispositif de morcellement positionné au niveau de l'extrémité distale de l'élément tubulaire (8).

10. Système selon la revendication 9, dans lequel le dispositif de morcellement est constitué par un ensemble d'électrodes électrochirurgicales bipolaires comprenant des première et deuxième électrodes, la première électrode étant positionnée au niveau de l'extrémité distale de l'élément tubulaire de sorte que, lorsqu'une tension de coupe électrochirurgicale est appliquée sur l'ensemble d'électrodes, le dispositif de traction de tissu peut être déplacé pour tirer le tissu contre l'extrémité distale de l'élément tubulaire (8) afin de former une âme de tissu coupé à l'intérieur de l'élément tubulaire, et peut en outre être déplacé afin de retirer le tissu coupé du site de l'opération chirurgicale.

11. Système chirurgical comprenant un boîtier (8, 24), un instrument chirurgical (14, 21, 22) et un ensemble d'étanchéité (23), l'instrument chirurgical pouvant être positionné à l'intérieur du boîtier (8, 24), le système pouvant être utilisé pour une chirurgie dans laquelle un site d'opération chirurgicale est alimenté avec du gaz sous pression, le boîtier ayant une extrémité proximale pouvant être positionnée à l'extérieur du corps d'un patient, l'instrument chirurgical ayant une extrémité distale pouvant être positionnée au niveau du site d'opération chirurgicale, et l'ensemble d'étanchéité (23) étant prévu au niveau de l'extrémité proximale du boîtier afin de réaliser l'étanchéité du boîtier par rapport à la partie d'extrémité proximale de l'élément chirurgical pour empêcher la fuite du gaz du site d'opération chirurgicale par l'extrémité proximale du boîtier, dans lequel l'ensemble d'étanchéité (23) est constitué par un diaphragme (26) dont les parties de bord périphérique (26a") sont fixées sur le boîtier, la partie centrale (26b") du diaphragme étant formée avec au moins une fente (27) à travers laquelle l'instrument chirurgical passe, l'ensemble d'étanchéité (23) étant tel que le mouvement de l'instrument chirurgical par rapport au boîtier amène le diaphragme (26) à se déplacer entre des première et deuxième positions opérationnelles dans lesquelles la fente est respectivement orientée vers et à distance de l'extrémité proximale du boîtier et de sorte que les bords de la fente se mettent en prise de manière étanche avec l'instrument chirurgical dans les première et deuxième positions opérationnelles, et dans toutes les positions entre elles, dans lequel la partie centrale du diaphragme est constituée par un élément laminaire fin (26b") positionné de manière centrale à l'intérieur d'une partie épaisse de bord circonférentiel (26a") du diaphragme, l'élément laminaire fin (26b") étant raccordé à la partie de bord circonférentiel (26a") par une section annulaire (29) qui est plus fine que l'élément laminaire (26b") de sorte qu'il empêche la force agissant sur la partie centrale d'être transférée à la partie de bord périphérique du diaphragme (26).

12. Système selon la revendication 11, dans lequel le diaphragme (26) comprend une partie interne (26b") ayant une première épaisseur transversale, une partie externe (26a") comprenant une deuxième épaisseur transversale, la première épaisseur transversale étant inférieure à la deuxième épaisseur transversale.
